# EUROPEAN PATENT SPECIFICATION

(11) **EP 0 522 491 B1**
(45) Date of publication and mention of the grant of the patent: **18.11.1998**
(21) Application number: 92111469.0
(22) Date of filing: 07.07.1992
(51) Int. Cl.: A61K 47/36, A61K 47/42

(54) **Compositions based on hyaluronic acid**
Arzneimittel auf Basis von Hyaluronsäure
Compositions à base d'acide hyaluronique

(30) Priority: 10.07.1991 JP 170205/91
(43) Date of publication of application: 13.01.1993
(73) Proprietor: TAKEDA CHEMICAL INDUSTRIES, LTD., Chuo-ku, Osaka 541 (JP)
(72) Inventor: Igari, Yasutaka, Higashinada-ku, Kobe Hyogo 658 (JP); Yamada, Minoru, Kawanishi, Hyogo 666-01 (JP); Ogawa, Yasuaki, Otokuni-gun, Kyoto 618 (JP)
(74) Representative: von Kreisler, Alek, Dipl.-Chem.

(56) References cited:
- WO-A-91/04058
- DATABASE: WPIL, AN=90-047916 (07), Derwent Publications Ltd, London, GB; & JP- A-2 000 213 (TAIHO PHARM. K.K.) 05-01-1990
- DATABASE: WPIL, AN=90-004036 (01), Derwent Publications Ltd, London, GB; & JP- A-1 287 041 (ROHMAN KOGYO K.K.) 17-11-1989
- DATABASE: WPIL, AN=91-054446 (08), Derwent Publications Ltd, London, GB; & JP-A-3 004 790 (KANEBO K.K.) 10-01-1991

## Description

### FIELD OF THE INVENTION

The present invention relates to a water-soluble composition which is of value as a sustained-release preparation of a pharmacologically active substance.

### BACKGROUND OF THE INVENTION

An object in formulating sustained-release preparations of a pharmacologically active substance, in particular a medicinal peptide, is to maintain the body fluid level of the peptide or the like within a certain effective range over a prolonged period of time. According to the mechanism of manifestation of its pharmacological effect, the peptide or the like may belong to either of the following two classes.
a) The pharmacological effect is not so dependent on the effective body fluid level and, even when the effect is produced to an excessive extent, the living body will not be adversely affected thereby.
b) The pharmacological effect is dependent on the effective body fluid level and abrupt or excessive manifestation of the pharmacological effect is harmful to the living body, so that the dose should be adjusted at regular intervals.

In Japanese Patent Application Laid-open No. 213/1990 is directed to sustained-release preparations containing hyaluronic acid or a nontoxic salt thereof together with a physiologically active peptide. In the examples described therein, a sustained-release calcitonin or elcatonin preparation containing sodium hyaluronate in a concentration of 5% was subcutaneously administered to male rats. Reduction in blood calcium level lasted at least 12 hours. Similarly, in male rats, after administration of a sustained-release human growth hormone preparation containing sodium hyaluronate in a concentration of 5%, the human growth hormone could be detected in the blood over at least 12 hours. In these cases, the duration of activity was evidently prolonged as compared with the control free of sodium hyaluronate. On the other hand, in another comparative example, a tegafur preparation containing sodium hyaluronate in a concentration of 5%, subcutaneously administered to male rats, did not show any prolonged action.

Meanwhile, these inventions make use of retarded diffusion, caused by the viscosity of hyaluronic acid, of a substance in solution at the site of administration. A cationic group-containing substance may undergo ion exchange with the carboxyl-containing hyaluronic acid macromolecule and this ion exchange can further retard the diffusion of the substance. According to Japanese Patent Application Laid-open No. 213/1990, the most preferred concentration of hyaluronic acid itself is 3 to 7%. However, once bubbles are formed due to the high viscosity, elimination thereof becomes a serious problem; centrifugation or application of a reduced pressure will be required for deaeration. At the same time, that high viscosity makes it necessary to use a large-gauge needle and the pain caused in patients is not negligible. Furthermore, even a fairly increased concentration of hyaluronic acid may fail in some instances to produce a prolonged effect, as in the example where tegafur was used.

Japanese Patent Application Laid-open No. 287041/1989 describes an example in which hyaluronic acid was used at a concentration of 1%. The caution in use for Artz Injection (Kaken Pharmaceutical, Japan), which is such a 1% sodium hyaluronate solution for intraarticular injection, recommends the use of a rather large-gauge needle of about 18-20G. As an injectable preparation for subcutaneous administration, such preparation causes much pain in patients.

Japanese Patent Application Laid-open No.129226/1987 (which corresponds to EP-224 987) describes that the hyaluronic acid concentration in injectable preparations may be within the range of about 0.05% to 4% (by weight) and, in some cases, may be higher depending on the final use of the preparations, without any mention of the applicability of such high concentrations to specific injectable preparations. It can be readily estimated, however, that any prolonged effect of a pharmacologically active substance will not be produced without using hyaluronic acid at a relatively high concentration, as mentioned above. There is indeed the possibility that the effect of a pharmacologically active substance could be prolonged depending on the viscosity of high-concentration hyaluronic acid and that a prolonged effect of a pharmacologically active substance could be similarly produced by utilizing the fact that the hyaluronic acid viscosity can be increased by lowering the pH into the acidic range (e.g. about 2.5). However, the higher the hyaluronic acid viscosity, the more difficult is the administration as an injection. In addition, it is feared that as the pH of the hyaluronic acid solution deviates from the physiological pH range, the stability of the pharmacologically active substance might be affected and/or the tissue at the administration site be damaged.

Japanese Patent Application Laid-open No. 4790/1991 describes that a protein like substance and polysaccharide are added to an aqueous solution of a protease produced by Bacillus licheniformis to stabilize the protease. However, there is no teaching or suggestion on the sustained-release of a polypeptide secreted in an animal body or its derivative and a chemically synthesized pharmacologically active substance.

Further, EP-A-0 503 583 (relevant under Art. 54(3), (4) EPC) discloses a sustained release pharmaceutical composition comprising (1) erythropoietin, (2) hyaluronic acid or its non-toxic salt and (3) a pharmaceutically acceptable carrier, diluent or excipient.

Although, as mentioned above, attempts have been made to use hyaluronic acid for making up pharmacologically active substances into sustained-release preparations, problems remain to be solved as to the adaptability or compatibility at the time of administration to living bodies.

### SUMMARY OF THE INVENTION

The present inventors made intensive investigations in an attempt to solve the above problems and, as a result, found that water-soluble compositions containing a pharmacologically active substance, a water-soluble species of hyaluronic acid and a water-soluble protein injectable into body fluids without showing any substantial pharmacological activity provide, when administered, a prolonged pharmacological effect without loss in the pharmacological activity of the active substance and with suppression of abrupt manifestation of the pharmacological effect at the initial stage following administration. Based on this finding, they made continued investigations and have now completed the present invention.

Thus the invention provides:
(1) a water-soluble composition which comprises
   (a) a pharmacologically active polypeptide having a molecular weight of 200 to 200 000, being secreted by an animal body and being selected from cytokines, peptide hormones, growth factors, factors affecting the cardiovascular system, factors affecting the central and peripheral nerve system, factors affecting body fluid electrolytes and blood organic constituents, factors affecting the bone and skeletal system, factors affecting the gastro-intestinal system, factors affecting the immune system, factors affecting the respiratory system, factors affecting the genital system, muteins, derivatives, analogs, homologs and active fragments thereof, except erythropoietin, or
      a chemically synthesized pharmacologically active substance;
   (b) a water-soluble species of hyaluronic acid or its non-toxic salt; and
   (c) a water-soluble protein injectable into body fluids without showing any substantial pharmacological activity,
      wherein said composition sustains the release of the ingredient (a);
(2) a method for preparing a water-soluble composition, which comprises mixing
   (a) a pharmacologically active polypeptide having a molecular weight of 200 to 200 000, being secreted by an animal body and being selected from cytokines, peptide hormones, growth factors, factors affecting the cardiovascular system, factors affecting the central and peripheral nerve system, factors affecting body fluid electrolytes and blood organic constituents, factors affecting the bone and skeletal system, factors affecting the gastro-intestinal system, factors affecting the immune system, factors affecting the respiratory system, factors affecting the genital system, muteins, derivatives, analogs, homologs and active fragments thereof, except erythropoietin, or
      a chemically synthesized pharmacologically active substance;
   (b) a water-soluble species of hyaluronic acid or its non-toxic salt; and
   (c) a water-soluble protein injectable into body fluids without showing any substantial pharmacological activity,
      whereby the prepared composition sustains the release of the ingredient (a).

In the present invention, a pharmacologically active polypeptide, except erythropoietin, secreted by an animal body or its derivative or a chemically synthesized pharmacologically active substance is abbreviated as pharmacologically active substance for short.

### BRIEF DESCRIPTION OF THE DRAWINGS

Figure 1 shows the serum mutein drug levels, as found in Experimental Example 1.

Figure 2 shows the serum glucose levels, as found in Experimental Example 2.

Figure 3 shows the time course of absolute neutrophil counts in peripheral blood, as found in Experimental Example 3.

Figure 4 shows the serum drug levels, as found in Experimental Example 4.

Figure 5 shows the serum PTH levels, as observed in Experimental Example 6.

### DETAILED DESCRIPTION OF THE INVENTION

In the present invention, the pharmacologically active polypeptide secreted by an animal body means a secretory peptide or protein that is produced in an animal body, and secreted internally to blood stream (endocrine) or to surrounding body fluid (paracrine or autocrine) or externally to the outside of the body (exocrine). In addition, the pharmacologically active polypeptide in the present invention means one that is produced by an animal body either as a natural or genetically engineered occurrence.

In the present invention, animal body means an animal comprising a single eukaryotic cell, an animal comprising multiple eukaryotic cells, or a tissue or organ isolated from the animal.

Examples of said animal comprising a single eukaryotic cell include yeast. Examples of said animal comprising multiple eukaryotic cells include a vertebrate animal, an invertebrate animal. And examples of said tissue or organ isolated from the animal include a cancer cell line derived from human or mouse.

Examples of said vertebrate animal include mammals (e.g. human, rodent (e.g. rat, mouse), bovine, horse, sheep), birds (e.g. poultry (e.g. chicken)), fishes (e.g. salmon, tuna), reptile (e.g. snake). Examples of said invertebrate animal include leech, and spider.

In said animal, an animal comprising multiple eukaryotic cells is preferred, and specifically, human, snake, spider and leech are more preferred.

As said polypeptide, one whose partition coefficient measured in octanol over water is below 0.1 is preferred, and one whose partition coefficient measured in octanol over water is below 0.05 is more preferred.

Said polypeptide has a molecular weight of 200 to 200000, with one having molecular weight of 300 to 90000 being preferred.

Said polypeptides may be naturally derived ones or ones produced by recombination technique or chemical synthesis.

Examples of cytokines include lymphokines, monokines and haematopoietic factors.

Examples of lymphokines include interferons (e.g. alpha, beta, gamma) and interleukins (e.g. IL-2 to IL-11).

Examples of monokines include IL-1, tumor necrotizing factor (e.g. TNF alfa and beta), and leukemic inhibitory factors (LIF).

Examples of haematopoietic factors include, granulocyte colony stimulating factors (G-CSF), granulocyte-macrophage colony stimulating factors (GM-SCF), macrophage colony stimulating factors (M-CSF).

Said haematopoietic factors also include ones that have thrombopoietic growth action, e.g., lymphocyte growth factor formulation (Leukoprol, Morinaga Milk, Japan), thrombopoietin, thrombocyte poiesis stimulatory factor, and megakaryocyte potentiator.

Examples of the factors that affect bone and skeletal system include calcitonin, bone Gla peptide, parathyroid hormone or its active fragment, parathyroid hormone related peptide (PTH-rp) or its active fragment (osteostatin, Endocrinology, 129, 324 (1991)) or histone H4-related osteogenic growth peptide (OGP) (The EMBO Journal, 11, 1867(1992)) or muteins of any of them, or derivatives or analogs of any of them.

Examples of growth factors include nerve growth factor (NGF), epidermal growth factor (EGF), fibrobalst growth factor (FGF), insulin like growth factor (IGF), transforming growth factor (TGF), and platelet derived growth factor (PDGF).

Examples of peptide hormone include insulin, growth hormone, luteinizing hormone releasing hormone (LH-RH), adrenocorticotropic hormone (ACTH), amylin, oxytosin, and leutenizing hormone (LH) or derivatives of any of them.

Examples of the factors that affect cardiovascular system include those that control blood pressure and arteriosclerosis such as endothelin, endothelin inhibitor, vasopressin, renin, angiotensin I, angiotensin II, angiotensin III, angiotensin I inhibitor, angiotensin II receptor antagonist and atrial natriuretic peptide (ANP), and antiarrhythmic peptide.

Examples of the factors that affect central and peripheral nerve system include opoid peptides (e.g., enkephalin, endorphin, kyotorphin), neurotropic factor (NTF), calcitonin gene related peptide, pituitary adenylate cyclase activating polypeptide (PACAP), thyrotropin releasing hormone (TRH) and salts and derivatives thereof (Japanese Patent Application Laid-open 121273/1975 corresponding to U.S. Patent No. 3,959,247 and No. 116465/1977 corresponding to U.S. Patent No. 4,100,152), and neurotensin.

Examples of the factors that affect gastro-intestinal system include secretin and gastrin.

Examples of the factors that affect immune system include those that control inflammation and malignant neoplasm and that attacks infective microorganisms.

Examples of the factors that affect body fluid electrolytes and blood organic constituents include those that control blood clotting, plasma cholesterol concentration, and metal ion concentration.

These peptides or factors may further include soluble receptors for the polypeptides.

Examples of cell attachment factors include laminin and inter cellular adhesion molecule (ICAM) 1.

Examples of the polypeptides include natural derived, synthetic or genetically engineered peptides and proteins capable of serving as antigens, e.g., cedar pollen and ragweed pollen. These are administered alone, in a hapten-bound form, or together with an adjuvant in compositions of the present invention, in the form of injections.

These peptides or factors each may include those chemically modified with synthetic polymer such as polyethyleneglycol, natural polymers such as chondroitin, saccharides, or non-peptide substances. Said non-peptide substances may be either ligands for receptors or antigens for antibodies. In addition, the polypeptides or factors each may include hybrid peptides in which multiple peptides are combined by chemical method or by recombination technique.

Among said polypeptides, interferons, interleukins, granulocyte colony stimulating factor (G-CSF), granulocyte-macrophage colony stimulating factor (GM-CSF), macrophage colony stimulating factor (M-CSF), fibroblast growth factor (FGF), tumor necrotizing factor (TNF), parathyroid hormone (PTH), calcitonin, insulin and luteinizing hormone-releasing hormone (LH-RH) are more preferred.

These peptides or factors each may include muteins differing in carbohydrate chain structure, factors having no carbohydrate chain, analogs or homologs, derivatives thereof, and active fragments derived from these, irrespective of their mechanisms of action, whether antagonistic or agonistic.

Examples of the chemically synthesized pharmacologically active substances include those that affect cardiovascular system, those that affect cell attachment, those that affect central and peripheral nerve system, those that affect body fluid electrolytes and blood organic constituents, those that affect bone and skeletal system, those that affect gastro-intestinal system, those that affect nephro-urinary system, those that affect connective tissues and skins, those that affect sensory organs, those that affect immune system, those that affect respiratory system, those that affect infective microorganisms, and those that affect genital system.

As the chemically synthesized pharmacologically active substances, those that affect cardiovascular system, those that affect cell attachment, those that affect central and peripheral nerve system, those that affect body fluid electrolytes and blood organic constituents, those that affect bone and skeletal system, those that affect gastro-intestinal system, those that affect immune system, and those that affect infective microorganisms are preferred.

Examples of the chemically synthesized pharmacologically active substances that affect cardiovascular system include anti-hypertensive agents such as calcium antagonist, angiotensin converting enzyme inhibitor, angiotensin II receptor antagonist and potassium channel opener.

Examples of the chemically synthesized pharmacologically active substances that affect cell attachment include RGD (arginine-glysine-asparaginic acid) antagonist.

Examples of the chemically synthesized pharmacologically active substances that affect central and peripheral nerve system include psychopharmaceuticals and antipsychotic agents such as diazepam, anti nausea-vomitting agent and anti-dimentia agents such as benzoquinon derivatives.

Examples of the chemically synthesized pharmacologically active substances that affect body fluid electrolytes and blood organic constituents include those that control blood clotting and plasma cholesterol concentration such as sodium pravastatin.

Examples of the chemically synthesized pharmacologically active substances that affect bone and skeletal system include anti osteoporosis agents such as bis-phosphonates, ipuriflavon, vitamin D3, and antirheumatic agents.

Examples of the chemically synthesized pharmacologically active substances that affect gastro-intestinal system includes proton pump inhibitor such as lansoprazol and H₂ receptor antagonist such as cimetidine.

Examples of the chemically synthesized pharmacologically active substances that affect immune system include anti-inflammatory agents such as indomethacin, anti tumor agents such as cis-platinum, adriamycin, anti-allergic agents and nonpeptide antigenic substance.

Examples of the chemically synthesized pharmacologically active substances that affect respiratory system include antiasthmatic agent.

Examples of the chemically synthesized pharmacologically active substances that affect infective microorganisms include antiviral agents and antibiotics such as cephalosporin and penicillin.

Examples of the chemically synthesized pharmacologically active substances that affect genital system include sex steroids such as testosteron.

Examples of the chemically synthesized pharmacologically active substances further include hormone (dexamethasone, predonisolone) and non-peptide antigenic substances.

Said chemically synthesized pharmacologically active substances include inorganic and organic ones.

In addition, these may include prostaglandins, leukotrients, saccharides, polysaccharides, metal chelates, or nucleotides such as antisense RNA or DNA.

Said chemically synthesized nonpeptide antigenic substances are administered alone, in a hapten-bound form, or together with an adjuvant in compositions of the present invention, in the form of injections.

In the present specification, the term "hyaluronic acid" includes hyaluronic acid which is a mucopolysaccharide composed of N-acetylglucosamine and glucuronic acid, nontoxic salts thereof, hyaluronic acid derivatives and nontoxic salts thereof. The nontoxic salts are, for example, salts with alkali metals, such as sodium and potassium, and salts with alkaline earth metals, such as magnesium and calcium. Among them, the sodium salt is most preferred. Hyaluronic acid and salts thereof preferably have a molecular weight of 1 x 10⁴ to 5 x 10⁶ (as determined by the viscosity method using intrinsic viscosity and the Mark-Houvinc equation), more preferably 1 x 10⁵ to 3 x 10⁶.

The water-soluble protein injectable into body fluids without showing any substantial pharmacological activity, which is to be incorporated in the composition of the present invention and which is hereinafter sometimes referred to briefly as "water-soluble protein", includes, among others, serum albumin, globulin, collagen, and gelatin. Among them, serum albumin is preferred. The water-soluble protein is preferred intact with no chemical modification.

The term "pharmacological effect" is defined herein as a substantial pharmacological effect caused by water-soluble protein alone at a concentration used in a single dosage unit form.

The water-soluble composition of this invention is adjusted such that when it is dissolved in water, the aqueous solution has a viscosity of not more than 500 mPa·s (cp), preferably within the range of 50 to 400 mPa·s (cp). Said viscosity depends on the molecular weight and concentration of hyaluronic acid, the concentration of the water-soluble protein, the concentration of the pharmacologically active substance and the concentration of the salt or salts such as sodium chloride (used as the isotonizing agent to be mentioned later herein), among others, and also depends to some extent on the pH of the water-soluble composition. Therefore, conditions adequate to give the desired water-soluble composition a viscosity within the above range should be selected.

The viscosity referred to herein is measured at a temperature of 25°C using a viscometer of the type E (TOKIMEC, Japan) with an LD cone.

A preferred range of hyaluronic acid concentration and a preferred range of water-soluble protein concentration in administering the water-soluble composition are mentioned below.

The water-soluble hyaluronic acid concentration at the time of administration should preferably be 0.01 to 3% (weight to volume), more preferably 0.05 to 2% (w/v).

When serum albumin, for instance, is used as the water-soluble protein in the composition of the invention, the serum albumin concentration at the time of administration should preferably be 0.001 to 5% (w/v), more preferably 0.005 to 2% (w/v). When globulin is used as the water-soluble protein, the globulin concentration at the time of administration should preferably be 0.001 to 5% (w/v), more preferably 0.005 to 2% (w/v). When collagen is used as the water-soluble protein, the collagen concentration at the time of administration should preferably be 0.001 to 1% (w/v), more preferably 0.005 to 0.2% (w/v). When gelatin is used as the water-soluble protein, the gelatin concentration at the time of administration should preferably be 0.001 to 1% (w/v), more preferably 0.005 to 0.5% (w/v).

The pH of a solution prepared from the water-soluble composition of the present invention should be such that said pH will not exert an adverse influence on the activity of the pharmacologically active substance but is within an acceptable range for injections in general and further such that said pH will neither cause a great change in viscosity of the solution nor allow formation of a precipitate or the like. Thus the solution should preferably have a pH of 4 to 8, more preferably a pH of 5 to 8.

As regards the proportion of the pharmacologically active substance in the composition, said substance may be contained therein in an effective amount which may vary depending on the activity of said substance and the therapeutic dose thereof. Generally, the weight ratio between the pharmacologically active substance and hyaluronic acid is preferably from 0.0001:1 to 10:1, preferably 0.0002:1 through 5:1, more preferably 0.0002:1 through 1:1, still more preferably 0.0002:1 through 0.1:1.

Although any particular mention cannot be made of the proportion of the water-soluble protein, either, said protein can be added in an amount generally employed in injectable pharmaceutical compositions and a preferred weight ratio between the water soluble protein and hyaluronic acid is 0.001:1 to 100:1, more preferably 0.01:1 through 10:1, still more preferably 0.1:1 through 10:1.

All the three essential components of the composition of the present invention, namely the pharmacologically active substance, hyaluronic acid and water-soluble protein, are preferably made to occur in a single unit dosage form. Thus, for example, the three are made to occur in an ampule or vial by dissolving or suspending them in sterile water or sterile physiological saline. In this case, the method of preparation may comprise admixing a solution of the pharmacologically active substance, a solution of hyaluronic acid and a solution of the water-soluble protein, or adding hyaluronic acid and the water-soluble protein, each in a powder form, to a solution of the pharmacologically active substance, or any other combination of adequate procedures. The dosage form may also be prepared by adding sterile water or sterile physiological saline to a lyophilizate or vacuum-dried powder in which the pharmacolgically active substance, hyaluronic acid and water-soluble protein coexist. This unit dosage form may further contain one or more of conventional additives such as pH adjusting agents (e.g. glycine, hydrochloric acid, sodium hydroxide), local anesthetics (e.g. xylocaine hydrochloride, chlorobutanol), isotonizing agents (e.g. sodium chloride, mannitol, sorbitol), and adsorption inhibitors (e.g. Tween® 80).

This unit dosage form may further contain pharmaceutically acceptable excipients such as polyethylene glycol 400 or dextran.

The goal of admixing the ingredients should be such that the activity of the pharmacologically active substance is maintained and bubble formation is minimized during the process. The ingredients are put into a vessel (for example bottle or drum) either at the same time or in any order. The total volume of the ingredients should be at most three quarters, preferrably, three fifths, more preferably half, still more preferably one third of the capacity of the vessel. The vessel is shaked gently and preferably rotated about its longitudinal axis for rotary blending. The number of revolution is selected according to the capacity of the vessel, the total volume of the ingredients, the concentration of hyaluronic acid or its non toxic salt, and so on. The preferred number of revolution ranges from 10 round per minute (rpm) through 1000 rpm, although this is not an exclusive range. The atmosphere in the vessel can be sterile clean air or aterile clean nitrogen gas. The resultant solution can be transferred to small vials or ampules, and can be further subjected to lyophilization.

The water-soluble composition of the present invention preferably takes the form of a parenteral preparation.

As said parenteral preparation, there may be mentioned injectable solutions, solutions for transmucosal administration, nasal solutions, otic solutions, etc.

Said injectable solutions include solutions for intravenous administration, for subcutaneous administration, for intraarticular administration, for intramuscular administration and for intraocular administration, among others. Since the viscosity of these long-acting preparations is adjusted to about 500 cp or less, the preparations can be readily drawn from ampules or vials into syringes using a 25G or 26G needle. Bubbles formed upon the drawing can be readily removed by a short period of mere standing.

The liquid form of the composition of the present invention filled in a soft capsule or the lyophilizate powder of the composition of the present invention filled in a hard capsule or compressed to tablet form may be administered to stomach, large intestine, or rectum.

The composition of the present invention may be in a form dissolved in water or in a lyophilized form with a crystalizing solute such as mannitol.

Addition of sterile water or sterile physiological saline to the lyophilizate gives an aqueous solution.

When the composition is in the form of a lyophilizate, it is required that the viscosity of and the component concentrations in the aqueous solution derived therefrom should be within the respective ranges mentioned hereinbefore.

The liquid form or the lyophilizate powder form of the composition of the present invention dissolved or dispersed in a solution of biodegradable polymer such as poly(lactic-glycolic)acid copolymer, poly(hydroxybutyric acid), poly(hydroxybutyric-glycolic) acid copolymer, or the mixture of these can be formulated, for example, to films, microcapsules (microspheres), or nanocapsules (nanospheres) according to the well known methods.

In addition, the composition of the present invention encapsulated in liposomes comprising phospholipids, cholesterols or the derivatives of these according to the well known methods can be further dispersed in physiological saline or the hyaluronic acid solution dissolved in physiological saline.

Further, the composition of the present invention can be supplied in a prefilled syringe for self-administration, since the liquid formulation will not be subject to physical disturbances such as shaking due to its low visous nature.

The composition of the present invention may be maintained at normal temperature or at normal refrigiration range, preferably 0°C to a room temperature (e.g. 25°C), more preferably from +2°C to +8°C.

The doses, target patients and target diseases of the composition of the present invention correspond to those of the pharmacologically active substance.

The water-soluble composition of the present invention is excellent in producing a prolonged effect. Even a low concentration of hyaluronic acid can produce the effect to a satisfactory extent. As a result, a small-gauge needle can be used, whereby pain in patients can be reduced. The composition has a low viscosity and therefore the possibility of bubble formation is much reduced. Thus the composition can be used with ease in clinical practice.

The following working examples and test examples are further illustrative of the present invention but are by no means limitative of the scope of the invention.

### Example 1 Sustained-release preparation containing basic fibroblast growth factor muteine

To 0.36 milliliter of a solution of recombinant human basic FGF muteine CS23 (hereinafter sometimes referred to as rhbFGF muteine CS23) (EP-281,822) (0.96 milligram protein/milliliter) were added 1.26 milliliters of physiological saline for injection and 8.1 milligrams of sodium hyaluronate (average molecular weight 1,470,000 daltons; Genzyme, U.S.A.), followed by further addition of 6 microliters of Albumin Nichiyaku (Nihon Pharmaceutical, Japan), a 20% solution of human serum albumin. Viscosity: 366 mPa·s (cp).

### Example 2

To 0.7 milliliter of physiological saline containing 10 micrograms of nerve cell growth factor (NGF) (Biomedical Technology, U.S.A.) was added 4 microliters of Albumin Nichiyaku (Nihon Pharmaceutical) containing human serum albumin at a concentration of 20%, followed by further addition of 1.4 milliliters of physiological saline for injection (Fuso Yakuhin, Japan) to make the whole volume 2.1 milliliters. To this solution was added 10.5 milligrams of sodium hyaluronate (average molecular weight 1,470,000 daltons; Genzyme). Viscosity: 285 mPa·s (cp).

### Example 3

To 0.7 milliliter of physiological saline containing 10 micrograms of epithelial growth factor (EGF) (Chemi-Con International, U.S.A.) was added 4 microliters of Albumin Nichiyaku (Nihon Pharmaceutical) containing human serum albumin at a concentration of 20%, followed by further addition of 1.4 milliliters of physiological saline for injection (Fuso Yakuhin) to make the whole volume 2.1 milliliters. To this solution was added 10.5 milligrams of sodium hyaluronate (average molecular weight 1,470,000 daltons; Genzyme). Viscosity: 305 mPa·s (cp).

### Example 4

To 0.7 milliliter of physiological saline containing 10 micrograms of insulin-like growth factor (IGF) (Chemi-Con International) was added 4 microliters of Albumin Nichiyaku (Nihon Pharmaceutical) containing human serum albumin at a concentration of 20%, followed by further addition of 1.4 milliliters of physiological saline for injection (Fuso Yakuhin) to make the whole volume 2.1 milliliters. To this solution was added 10.5 milligrams of sodium hyaluronate (average molecular weight 1,470,000 daltons; Genzyme). Viscosity: 298 mPa·s (cp).

### Example 5

To 0.7 milliliter of physiological saline containing 10,000 international units of interferon alpha (Lee Biomolecular Research Labo, U.S.A.) was added 4 microliters of Albumin Nichiyaku (Nihon Pharmaceutical) containing human serum albumin at a concentration of 20%, followed by further addition of 1.4 milliliters of physiological saline for injection (Fuso Yakuhin) to make the whole volume 2.1 milliliters. To this solution was added 10.5 milligrams of sodium hyaluronate (average molecular weight 1,470,000 daltons; Genzyme). Viscosity: 312 mPa·s (cp).

### Example 6

To 0.7 milliliters of physiological saline containing 10,000 international units of interferon beta (Pesel, Germany) was added 4 microliters of Albumin Nichiyaku (Nihon Pharmaceutical) containing human serum albumin at a concentration of 20%, followed by further addition of 1.4 milliliters of physiological saline for injection (Fuso Yakuhin) to make the whole volume 2.1 milliliters. To this solution was added 10.5 milligrams of sodium hyaluronate (average molecular weight 1,470,000 daltons; Genzyme). Viscosity: 320 mPa·s (cp).

### Example 7

To 0.7 milliliter of physiological saline containing 10,000 international units of interferon gamma (Genzyme, U.S.A.) was added 4 microliters of Albumin Nichiyaku (Nihon Pharmaceutical) containing human serum albumin at a concentration of 20%, followed by further addition of 1.4 milliliters of physiological saline for injection (Fuso Yakuhin) to make the whole volume 2.1 milliliters. To this solution was added 10.5 milligrams of sodium hyaluronate (average molecular weight 1,470,000 daltons; Genzyme). Viscosity: 311 mPa·s (cp).

### Example 8

To 0.7 milliliter of physiological saline containing 10 micrograms of interleukin 2 (IL-2) (produced by the method described in Japanese Patent Application Laid-open No. 78799/1986 corresponding to EP-176299 and purified by the method described in Japanese Patent Application Laid-open No. 115528/1985corresponding to EP-145390; mixture of N-terminal Met-containing species and N-terminal Met-free species) was added 4 microliters of Albumin Nichiyaku (Nihon Pharmaceutical) containing human serum albumin at a concentration of 20%, followed by further addition of 1.4 milliliters of physiological saline for injection (Fuso Yakuhin) to make the whole volume 2.1 milliliters. To this solution was added 10.5 milligrams of sodium hyaluronate (average molecular weight 1,470,000 daltons; Genzyme). Viscosity: 320 mPa·s (cp).

### Example 9

To 0.7 milliliter of physiological saline containing 1 microgram of transforming growth factor (TGF-β) (Wako Pure Chemical Industries, Japan) was added 4 microliters of Albumin Nichiyaku (Nihon Pharmaceutical) containing human serum albumin at a concentration of 20%, followed by further addition of 1.4 milliliters of physiological saline for injection (Fuso Yakuhin) to make the whole volume 2.1 milliliters. To this solution was added 10.5 milligrams of sodium hyaluronate (average molecular weight 1,470,000 daltons; Genzyme). Viscosity: 353 mPa·s (cp).

### Example 10

To 0.7 milliliter of physiological saline containing 10 micrograms of parathyroid hormone (PTH) (Bachem Fine Chemicals, Switzerland) was added 4 microliters of Albumin Nichiyaku (Nihon Pharmaceutical) containing human serum albumin at a concentration of 20%, followed by further addition of 1.4 milliliters of physiological saline for injection (Fuso Yakuhin) to make the whole volume 2.1 milliliters. To this solution was added 10.5 milligrams of sodium hyaluronate (average molecular weight 1,470,000 daltons; Genzyme). Viscosity: 251 mPa·s (cp).

### Example 11

To 0.7 milliliter of physiological saline containing 5,000 units of granulocyte-macrophage colony stimulating factor (GM-CSF) (ICN Biomedicals, U.S.A.) was added 4 microliters of Albumin Nichiyaku (Nihon Pharmaceutical) containing human serum albumin at a concentration of 20%, followed by further addition of 1.4 milliliters of physiological saline for injection (Fuso Yakuhin) to make the whole volume 2.1 milliliters. To this solution was added 10.5 milligrams of sodium hyaluronate (average molecular weight 1,470,000 daltons; Genzyme). Viscosity: 301 mPa·s (cp).

### Example 12

To 0.7 milliliter of physiological saline containing 5,000 units of macrophage colony stimulating factor (M-CSF) (ICN Biomedicals) was added 4 microliters of Albumin Nichiyaku (Nihon Pharmaceutical) containing human serum albumin at a concentration of 20%, followed by further addition of 1.4 milliliters of physiological saline for injection (Fuso Yakuhin) to make the whole volume 2.1 milliliters. To this solution was added 10.5 milligrams of sodium hyaluronate (average molecular weight 1,470,000 daltons; Genzyme). Viscosity: 299 mPa·s (cp).

### Example 13

To 0.7 milliliter of physiological saline containing 1 microgram of acid fibroblast growth factor (FGF-α) (Toyobo, Japan) was added 4 microliters of Albumin Nichiyaku (Nihon Pharmaceutical) containing human serum albumin at a concentration of 20%, followed by further addition of 1.4 milliliters of physiological saline for injection (Fuso Yakuhin) to make the whole volume 2.1 milliliters. To this solution was added 10.5 milligrams of sodium hyaluronate (average molecular weight 1,470,000 daltons; Genzyme). Viscosity: 274 mPa·s (cp).

### Example 14

To 1 microgram of tumor necrosis factor (TNF-α) (Wako Pure Chemical, Japan) was added 0.7 milliliter of physiological saline for injection (Fuso Yakuhin). To the mixture was added 4 microliters of Albumin Nichiyaku (Nihon Pharmaceutical) containing human serum albumin at a concentration of 20%, followed by further addition of 1.4 milliliters of physiological saline for injection (Fuso Yakuhin) to make the whole volume 2.1 milliliters. To this solution was added 10.5 milligrams of sodium hyaluronate (average molecular weight 1,470,000 daltons; Genzyme). Viscosity: 286 mPa·s (cp).

### Example 15

To 0.7 milliliter of physiological saline containing 400 units of Serratia-derived superoxide dismutase (SOD) (Japanese Patent Application Laid-open No. 29285/1982 corresponding to EP-45,222 and No. 16685/1983 corresponding to EP-70,656) was added 4 microliters of Albumin Nichiyaku (Nihon Pharmaceutical) containing human serum albumin at a concentration of 20%, followed by further addition of 1.4 milliliters of physiological saline for injection (Fuso Yakuhin) to make the whole volume 2.1 milliliters. To this solution was added 10.5 milligrams of sodium hyaluronate (average molecular weight 1,470,000 daltons; Genzyme). Viscosity: 291 mPa·s (cp).

### Example 16

To 0.7 milliliter of physiological saline containing 10 micrograms of vasopressin (Cambridge Research Biochemical, Great Britain) was added 4 microliters of Albumin Nichiyaku (Nihon Pharmaceutical) containing human serum albumin at a concentration of 20%, followed by further addition of 1.4 milliliters of physiological saline for injection (Fuso Yakuhin) to make the whole volume 2.1 milliliters. To this solution was added 10.5 milligrams of sodium hyaluronate (average molecular weight 1,470,000 daltons; Genzyme). Viscosity: 220 mPa·s (cp).

### Example 17

To 0.7 milliliter of physiological saline containing 10 micrograms of somatostatin (Bachem Fine Chemicals) was added 4 microliters of Albumin Nichiyaku (Nihon Pharmaceutical) containing human serum albumin at a concentration of 20%, followed by further addition of 1.4 milliliters of physiological saline for injection (Fuso Yakuhin) to make the whole volume 2.1 milliliters. To this solution was added 10.5 milligrams of sodium hyaluronate (average molecular weight 1,470,000 daltons; Genzyme). Viscosity: 249 mPa·s (cp).

### Example 18

To 0.7 milliliter of physiological saline containing 10 micrograms of oxytocin (Bachem Fine Chemicals) was added 4 microliters of Albumin Nichiyaku (Nihon Pharmaceutical) containing human serum albumin at a concentration of 20%, followed by further addition of 1.4 milliliters of physiological saline for injection (Fuso Yakuhin) to make the whole volume 2.1 milliliters. To this solution was added 10.5 milligrams of sodium hyaluronate (average molecular weight 1,470,000 daltons; Genzyme). Viscosity: 253 mPa·s (cp).

### Example 19

To 0.7 milliliter of physiological saline containing luteinizing hormone releasing hormone (LH-RH) (Bachem Fine Chemicals) 1 was added 4 microliters of Albumin Nichiyaku (Nihon Pharmaceutical) containing human serum albumin at a concentration of 20%, followed by further addition of 1.4 milliliters of physiological saline for injection (Fuso Yakuhin) to make the whole volume 2.1 milliliters. To this solution was added 10.5 milligrams of sodium hyaluronate (average molecular weight 1,470,000 daltons; Genzyme). Viscosity: 241 mPa·s (cp).

### Example 20

To 0.7 milliliter of physiological saline containing 10 micrograms of growth hormone releasing factor (Bachem Fine Chemicals) was added 4 microliters of Albumin Nichiyaku (Nihon Pharmaceutical) containing human serum albumin at a concentration of 20%, followed by further addition of 1.4 milliliters of physiological saline for injection (Fuso Yakuhin) to make the whole volume 2.1 milliliters. To this solution was added 10.5 milligrams of sodium hyaluronate (average molecular weight 1,470,000 daltons; Genzyme). Viscosity: 265 mPa·s (cp).

### Example 21

To 0.7 milliliter of physiological saline containing 10 micrograms of growth hormone (UCB Bioproducts, Belgium) was added 4 microliters of Albumin Nichiyaku (Nihon Pharmaceutical) containing human serum albumin at a concentration of 20%, followed by further addition of 1.4 milliliters of physiological saline for injection (Fuso Yakuhin) to make the whole volume 2.1 milliliters. To this solution was added 10.5 milligrams of sodium hyaluronate (average molecular weight 1,470,000 daltons; Genzyme). Viscosity: 293 mPa·s (cp).

### Example 22

To 0.7 milliliter of physiological saline containing 10 micrograms of calcitonin (UCB Bioproducts) was added 4 microliters of Albumin Nichiyaku (Nihon Pharmaceutical) containing human serum albumin at a concentration of 20%, followed by further addition of 1.4 milliliters of physiological saline for injection (Fuso Yakuhin) to make the whole volume 2.1 milliliters. To this solution was added 10.5 milligrams of sodium hyaluronate (average molecular weight 1,470,000 daltons; Genzyme). Viscosity: 238 mPa·s (cp).

### Example 23

To 0.7 milliliter of physiological saline containing 10 micrograms of calcitonin gene-related peptide (UCB Bioproducts) was added 4 microliters of Albumin Nichiyaku (Nihon Pharmaceutical) containing human serum albumin at a concentration of 20%, followed by further addition of 1.4 milliliters of physiological saline for injection (Fuso Yakuhin) to make the whole volume 2.1 milliliters. To this solution was added 10.5 milligrams of sodium hyaluronate (average molecular weight 1,470,000 daltons; Genzyme). Viscosity: 229 mPa·s (cp).

### Example 24

To 0.7 milliliter of physiological saline containing 10 micrograms of brain natriuretic peptide (Peptide Institute, Japan) was added 4 microliters of Albumin Nichiyaku (Nihon Pharmaceutical) containing human serum albumin at a concentration of 20%, followed by further addition of 1.4 milliliters of physiological saline for injection (Fuso Yakuhin) to make the whole volume 2.1 milliliters. To this solution was added 10.5 milligrams of sodium hyaluronate (average molecular weight 1,470,000 daltons; Genzyme). Viscosity: 241 mPa·s (cp).

### Example 25

To 0.7 milliliter of physiological saline containing 10 micrograms of atrial natriuretic peptide (Cambridge Research Biochemicals, U.K.) was added 4 microliters of Albumin Nichiyaku (Nihon Pharmaceutical) containing human serum albumin at a concentration of 20%, followed by further addition of 1.4 milliliters of physiological saline for injection (Fuso Yakuhin) to make the whole volume 2.1 milliliters. To this solution was added 10.5 milligrams of sodium hyaluronate (average molecular weight 1,470,000 daltons; Genzyme). Viscosity: 243 mPa·s (cp).

### Example 26

To 0.7 milliliter of physiological saline containing 10 micrograms of an osterocalcin analog (American Peptide, U.S.A.) was added 4 microliters of Albumin Nichiyaku (Nihon Pharmaceutical) containing human serum albumin at a concentration of 20%, followed by further addition of 1.4 milliliters of physiological saline for injection (Fuso Yakuhin) to make the whole volume 2.1 milliliters. To this solution was added 10.5 milligrams of sodium hyaluronate (average molecular weight 1,470,000 daltons; Genzyme). Viscosity: 265 mPa·s (cp).

### Example 27

To 0.7 milliliter of physiological saline containing 10 micrograms of hepatocyte growth factor (Peptide Institute, Japan) was added 4 microliters of Albumin Nichiyaku (Nihon Pharmaceutical) containing human serum albumin at a concentration of 20%, followed by further addition of 1.4 milliliters of physiological saline for injection (Fuso Yakuhin) to make the whole volume 2.1 milliliters. To this solution was added 10.5 milligrams of sodium hyaluronate (average molecular weight 1,470,000 daltons; Genzyme). Viscosity: 271 mPa·s (cp).

### Example 28

To 0.7 milliliter of physiological saline containing urokinase (Nippon Chemical Research, Japan) was added 4 microliters of Albumin Nichiyaku (Nihon Pharmaceutical) containing human serum albumin at a concentration of 20%, followed by further addition of 1.4 milliliters of physiological saline for injection (Fuso Yakuhin) to make the whole volume 2.1 milliliters. To this solution was added 10.5 milligrams of sodium hyaluronate (average molecular weight 1,470,000 daltons; Genzyme). Viscosity: 311 mPa·s (cp).

### Example 29

To 0.7 milliliter of physiological saline containing 10 micrograms of tissue plasminogen activator (TPA) (Bioscot, Great Britain) was added 4 microliters of Albumin Nichiyaku (Nihon Pharmaceutical) containing human serum albumin at a concentration of 20%, followed by further addition of 1.4 milliliters of physiological saline for injection (Fuso Yakuhin) to make the whole volume 2.1 milliliters. To this solution was added 10.5 milligrams of sodium hyaluronate (average molecular weight 1,470,000 daltons; Genzyme). Viscosity: 335 mPa·s (cp).

### Example 30

To 0.7 milliliter of physiological saline containing 1 microgram of interleukin 3 (IL-3) (R&D Systems, U.S.A.) is added 4 microliters of Albumin Nichiyaku (Nihon Pharmaceutical) containing human serum albumin at a concentration of 20%, followed by further addition of 1.4 milliliters of physiological saline for injection (Fuso Yakuhin) to make the whole volume 2.1 milliliters. To this solution was added 10.5 milligrams of sodium hyaluronate (average molecular weight 1,470,000 daltons; Genzyme). Viscosity: 259 mPa·s (cp).

### Example 31

To 0.7 milliliter of physiological saline containing 1 microgram of interleukin 4 (IL-4) (R&D Systems) is added 4 microliters of Albumin Nichiyaku (Nihon Pharmaceutical) containing human serum albumin at a concentration of 20%, followed by further addition of 1.4 milliliters of physiological saline for injection (Fuso Yakuhin) to make the whole volume 2.1 milliliters. To this solution was added 10.5 milligrams of sodium hyaluronate (average molecular weight 1,470,000 daltons; Genzyme). Viscosity: 265 mPa·s (cp).

### Example 32

To 0.7 milliliter of physiological saline containing 1 microgram of interleukin 5 (IL-5) (Amgen, U.S.A.) is added 4 microliters of Albumin Nichiyaku (Nihon Pharmaceutical) containing human serum albumin at a concentration of 20%, followed by further addition of 1.4 milliliters of physiological saline for injection (Fuso Yakuhin, Japan) to make the whole volume 2.1 milliliters. To this solution was added 10.5 milligrams of sodium hyaluronate (average molecular weight 1,470,000 daltons; Genzyme). Viscosity: 282 mPa·s (cp).

### Example 33

To 0.7 milliliter of physiological saline containing 1 microgram of interleukin 6 (IL-6) (Amgen) is added 4 microliters of Albumin Nichiyaku (Nihon Pharmaceutical) containing human serum albumin at a concentration of 20%, followed by further addition of 1.4 milliliters of physiological saline for injection (Fuso Yakuhin) to make the whole volume 2.1 milliliters. To this solution was added 10.5 milligrams of sodium hyaluronate (average molecular weight 1,470,000 daltons; Genzyme). Viscosity: 249 mPa·s (cp).

### Example 34

To 0.7 milliliter of physiological saline containing 1 microgram of interleukin 7 (IL-7) (Genzyme) is added 4 microliters of Albumin Nichiyaku (Nihon Pharmaceutical) containing human serum albumin at a concentration of 20%, followed by further addition of 1.4 milliliters of physiological saline for injection (Fuso Yakuhin) to make the whole volume 2.1 milliliters. To this solution was added 10.5 milligrams of sodium hyaluronate (average molecular weight 1,470,000 daltons; Genzyme). Viscosity: 255 mPa·s (cp).

### Example 35

To 0.7 milliliter of physiological saline containing 1 microgram of interleukin 8 (IL-8) (Genzyme) is added 4 microliters of Albumin Nichiyaku (Nihon Pharmaceutical) containing human serum albumin at a concentration of 20%, followed by further addition of 1.4 milliliters of physiological saline for injection (Fuso Yakuhin) to make the whole volume 2.1 milliliters. To this solution was added 10.5 milligrams of sodium hyaluronate (average molecular weight 1,470,000 daltons; Genzyme). Viscosity: 263 mPa·s (cp).

### Example 36 Long-acting insulin preparation

Three milligrams of pig insulin (26.8 units/milligram; Diosynth, Netherlands) was dissolved in 1.5 milliliters of 0.1 N hydrochloric acid and then 1.65 milliliters of physiological saline for injection was added, followed by addition of 15.75 milligrams of sodium hyaluronate (average molecular weight 1,470,000 daltons; Genzyme). To the thus-obtained solution was added 4 microliters of Albumin Nichiyaku (Nihon Pharmaceutical) containing human serum albumin at a concentration of 20%. Viscosity: 384 mPa·s (cp).

### Example 37 Long lasting preparation containing granulocyte colony stimulating factor (G-CSF)

To 0.0466 milliliter of a human granulocyte colony stimulating factor preparation (Nupogen; Amgen, U.S.A.) containing, per milliliter, 300 micrograms of recombinant human G-CSF, 50 milligrams of mannitol and 0.004% of Tween 80 was added 4.154 milliliters of physiological saline for injection, followed by further addition of 21 milligrams of sodium hyaluronate (average molecular weight 1,470,000 daltons; Genzyme). To the thus-obtained solution was added 8 microliters of Albumin Nichiyaku (Nihon Pharmaceutical) containing human serum albumin at a concentration of 20%. Viscosity: 264 mPa·s (cp).

### Example 38 Long-acting insulin preparation

Three milligrams of pig insulin (26.8 units/milligram; Diosynth) was dissolved in 1.5 milliliters of 0.1 N hydrochloric acid and then 1.65 milliliters of physiological saline for injection was added, followed by further addition of 15.75 milligrams of sodium hyaluronate (average molecular weight 1,470,000 daltons; Genzyme). To the solution obtained was added 0.5 milligram of human immunoglobulin G (Cappel). Viscosity: 328 mPa·s (cp).

### Example 39 Long-acting granulocyte colony stimulating factor (G-CSF) preparation

To 0.0233 milliliters of a human granulocyte colony stimulating factor preparation (Nupogen; Amgen, U.S.A.) containing, per milliliter, 300 micrograms of a recombinant human G-CSF, 50 milligrams of mannitol and 0.004% of Tween® 80 was added 1.377 milliliters of physiological saline for injection, followed by further addition of 8 microliters of Albumin Nichiyaku (Nippon Seiyaku) containing 20% of human serum albumin. To the resultant mixture was added 0.7 milliliter of a 1.5% (w/v) solution of sodium hyaluronate (average molecular weight: 1,470,000 daltons; Genzyme, U.S.A.) in physiological saline for injection (Fuso Pharmaceutical). A glass vial (capacity: 5 milliliters) containing these solutions was tightly stoppered and rotated, for rotary blending, in a 200-milliliter eggplant-shaped flask equipped with a three-one motor (Heydon, Japan) about its longitudinal axis (20-100 revolutions per minute (rpm)) for one hour. The above procedure gave a long-acting dosing solution substantially free of bubbles.

### Example 40

To 0.023 milliliter of a human granulocyte colony stimulating factor preparation (Nupogen; Amgen) containing, per milliliter, 300 micrograms of a recombinant human G-CSF, 50 milligrams of mannitol and 0.004% of Tween 80 was added 1.377 milliliters of physiological saline for injection, followed by further addition of 4 microliters of Albumin Nichiyaku (Nippon Seiyaku) containing 20% of human serum albumin. To the resultant mixture was added 0.7 milliliter of a 1.5% (w/v) solution of sodium hyaluronate (average molecular weight: ca. 500,000 daltons) in physiological saline for injection (Fuso Pharmaceutical). A glass vial (capacity: 5 milliliters) containing these solutions was hermetically stoppered and subjected to rotary blending in a 200-milliliter eggplant-shaped flask equipped with a three-one motor (Heydon) by rotation (20-100 rpm) about its longitudinal axis for one hour. The above procedure gave a long-acting dosing solution substantially free of bubbles.

### Example 41

To 0.023 milliliter of a human granulocyte colony stimulating factor preparation (Nupogen; Amgen) containing, per milliliter, 300 micrograms of a recombinant human G-CSF, 50 milligrams of mannitol and 0.004% of Tween® 80 was added 1.039 milliliters of physiological saline for injection, followed by further addition of 4 microliters of Albumin Nichiyaku (Nippon Seiyaku) containing 20% of human serum albumin. To the resultant mixture was added 1.039 milliliters of a 1% solution of sodium hyaluronate (average molecular weight: ca. 800,000-900,000 daltons; Artz Injection; Seikagaku Corporation, Japan). A glass vial (capacity: 5 milliliters) containing these solutions was tightly stoppered and subjected to rotary blending in a 200-milliliter eggplant-shaped flask equipped with a three-one motor (Heydon) by rotation (20-100 rpm) about its longitudinal axis for one hour. The above procedure gave a long-acting dosing solution substantially free of bubbles.

### Example 42

To 0.023 milliliter of a human granulocyte colony stimulating factor preparation (Nupogen; Amgen) containing, per milliliter, 300 micrograms of a recombinant human G-CSF, 50 milligrams of mannitol and 0.004% Tween® 80 was added 1.377 milliliters of physiological saline for injection, followed by further addition of 4 microliters of Albumin Nichiyaku (Nippon Seiyaku) containing 20% of human serum albumin. To the resultant mixture was added 0.7 milliliter of a 1.2% (w/v) solution of sodium hyaluronate (average molecular weight: 1,800,000 daltons; Genzyme) in physiological saline for injection (Fuso Pharmaceutical). A glass vial (capacity: 5 milliliters) containing these solutions was tightly stoppered and subjected to rotary blending in a 200-milliliter eggplant-shaped flask equipped with a three-one motor (Heydon) by rotation (20-100 rpm) about its longitudinal axis for one hour. The above procedure gave a long-acting dosing solution substantially free of bubbles.

### Example 43

To 0.023 milliliter of a human granulocyte colony stimulating factor preparation (Nupogen; Amgen) containing, per milliliter, 300 micrograms of a recombinant human G-CSF, 50 milligrams of mannitol and 0.004% of Tween® 80 was added 1.377 milliliters of physiological saline for injection, followed by further addition of 4 microliters of Albumin Nichiyaku (Nippon Seiyaku) containing 20% of human serum albumin. To the resultant mixture was added 0.7 milliliter of a 0.9% (w/v) solution of sodium hyaluronate (average molecular weight: 2,300,000 daltons; Genzyme) in physiological saline for injection (Fuso Pharmaceutical). A glass vial (capacity: 5 milliliters) containing these solutions was hermetically stoppered and subjected to rotary blending in a 200-milliliter eggplant-shaped flask equipped with a three-one motor (Heydon) by rotation (20-100 rpm) about its longitudinal axis for one hour. The above procedure gave a long-acting dosing solution substantially free of bubbles.

### Example 44

In 1 milliliter of physiological saline for injection was dissolved 300 micrograms of an active human parathyroid hormone (PTH) fragment (Bachem Fine Chemicals, Switzerland) covering from the amino terminal to the 34th amino acid of PTH, followed by addition of 4 microliters of Albumin Nichiyaku (Nippon Seiyaku) containing 20% of human serum albumin. To the resultant mixture was added 1.5 milliliters of a 1.2% (w/v) solution of sodium hyaluronate (average molecular weight: ca. 1,800,000 daltons; Genzyme) in physiological saline (Fuso Pharmaceutical). A glass vial (capacity: 10 milliliters) containing these solutions was subjected to rotary blending in a 200 milliliter eggplant-shaped flask equipped with a three-one motor (Heydon) by rotation (20-100 rpm) about its longitudinal axis for one hour. The above procedure gave a long-acting dosing solution substantially free of bubbles.

### Example 45

In 1 milliliter of physiological saline for injection was dissolved 300 micrograms of human parathyroid hormone (PTH) (Bachem Fine Chemicals), followed by addition of 4 microliters of Albumin Nichiyaku (Nippon Seiyaku) containing 20% of human serum albumin. To the resultant mixture was added 1.5 milliliters of a 1.2% (w/v) solution of sodium hyaluronate (average molecular weight: 1,800,000 daltons; Genzyme) in physiological saline for injection (Fuso Pharmaceutical). A glass vial (capacity: 10 milliliters) containing these solutions was tightly closed and subjected to rotary blending in a 200-milliliter eggplant-shaped flask equipped with a three-one motor (Heydon) by rotation (20-100 rpm) about its longitudinal axis for one hour. The above procedure gavea long-acting dosing solution substantially free of bubbles.

### Example 46

To a solution containing 100 ATU of hirudin (Peninsula Laboratories Inc., U.S.A.) is added physiological saline for injection to make the whole volume 1.4 milliliters. Then, 4 microliters of Albumin Nichiyaku (Nippon Seiyaku) containing 20% of human serum albumin is added. To the resultant mixture is added 0.7 milliliter of a 1.2% (w/v) solution of sodium hyaluronate (average molecular weight: 1,800,000 daltons; Genzyme) in physiological saline (Fuso Pharmaceutical). A glass vial (capacity: 5 milliliters) containing these solutions is tightly stoppered and subjected to rotary blending in a 200-milliliter eggplant-shaped flask equipped with a three-one motor (Heydon) by rotation (20-100 rpm) about its longitudinal axis for one hour. The above procedure gives a long-acting dosing solution substantially free of bubbles.

### Example 47

In 1.4 milliliters of physiological saline was dissolved 10 milligrams of thyrotropic hormone releasing hormone (TRH; Bachem Fine Chemicals, Switzerland), followed by addition of 4 microliters of Albumin Nichiyaku (Nippon Seiyaku) containing 20% of human serum albumin. To the resultant mixture was added 0.7 milliliter of a 1.2% (w/v) solution of sodium hyaluronate (average molecular weight: 1,800,000 daltons; Genzyme) in physiological saline for injection (Fuso Pharmaceutical). A glass vial (capacity: 5 milliliters) containing these solutions was hermetically stoppered and subjected to rotary blending in a 200-milliliter eggplant-shaped flask equipped with a three-one motor (Heydon) by rotation (20-100 rpm) about its longitudinal axis for one hour. The above procedure gave a long-acting dosing solution substantially free of bubbles.

### Example 48

In 1.4 milliliters of physiological saline was dissolved 10 milligrams of cefotiam dihydrochloride (Takeda Chemical Industries, Japan), followed by addition of 4 microliters of Albumin Nichiyaku (Nippon Seiyaku) containing 20% of human serum albumin. To the resultant mixture was added 0.7 milliliter of a 1.2% (w/v) solution of sodium hyaluronate (average molecular weight: ca. 1,800,000 daltons; Genzyme) in physiological salien for injection (Fuso Pharmaceutical). A glass vial (capacity: 5 milliliters) containing these solutions was tightly stoppered and subjected to rotary blending in a 200-milliliter eggplant-shaped flask equipped with a three-one motor (Heydon) by rotation (20-100 rpm) about its longitudinal axis for one hour. The above procedure gave a long-acting dosing solution substantially free of bubbles.

### Example 49

To each of two vials of Canferon-A300 (Takeda Chemical, Japan) containing 3 million international units (IU) of interferon alfa-2a and 5 mg of human serum albumin was added 1 ml of distilled water to provide an interferon alfa injection. 1.4 ml of the interferon alfa injection prepared as above (containing 4.2 million IU of interferon alfa-2a) and 0.7 ml of 1.5% (w/v) physiological saline solution of sodium hyaluronate (average molecular weight, 1,470,000 daltons) were put into a glass vial (capacity: 5 ml). The glass vial containing these solutions was hermetically closed and subjected to rotary blending in a 200 ml egg-plant-type flask equipped with a three-one motor (Heydon, Japan) by rotation about its longitudinal axis (20-100 rpm) for 1 hour. The procedure gave a long-acting dosing solution substantially free of bubbles.

### Example 50

To 0.4 milliliter of a solution of recombinant human basic FGF mutein CS23 (EP-281,822)(0.96 milligram protein/milliliter) were added 2.4 milliliters of physiological saline for injection and 15 microliters of Albumin Nichiyaku (Nihon Pharmaceutical, Japan), a 20% solution of human serum albumin, followed by addition of 1.4 milliliters of 1.5% (w/v) physiological saline solution of sodium hyaluronate (average molecular weight, 1470,000 daltons). A glass vial (capacity: 12 ml) was hermetically closed and subjected to rotary blending in a 200 ml egg-plant type flask equipped with a three-one motor (Heydon, Japan) by rotation about its longitudinal axis (20-100 rpm) for 1 hour. The procedure gave a long acting dosing solution substantially free of bubbles.

### Example 51

In 2 ml of physiological saline for injection (Fuso Pharmaceutical, Japan) was added 300 micrograms of parathyroid hormone (PTH) (Bachem Fine Chemicals). A glass vial (capacity: 5 ml) filled with the above injection was added 4 microliters of Nichiyaku Albumin (Nihon Seiyaku) containing 20% (v/v) of human serum albumin and 1 ml of a 1.5% (w/v) solution of sodium hyaluronate (average molecular weight 1,470,000; Genzyme) in physiological saline. The glass vial containing these solutions was hermetically closed and subjected to rotary blending by rotation about its longitudinal axis (20-100 rpm) in a three-one motor (Heydon; Japan) for one hour. The procedure gave a long-acting dosing solution substantially free of bubbles.

### Experimental Example 1

The following two injections were further prepared and subjected to testing.

### Comparative Preparation 1

To 0.35 milliliter of a solution of recombinant human basic FGF mutein CS23 (EP-281,822) (0.96 milligram protein/milliliter) was added 1.225 milliliters of physiological saline for injection.

### Comparative Preparation 2

To 0.36 milliliter of a solution of recombinant human basic FGF mutein CS23 (EP-281,822) (0.96 milligram protein/milliliter) were added 1.26 milliliters of physiological saline for injection and 8.1 milligrams of sodium hyaluronate (average molecular weight 1,470,000 daltons; Genzyme).

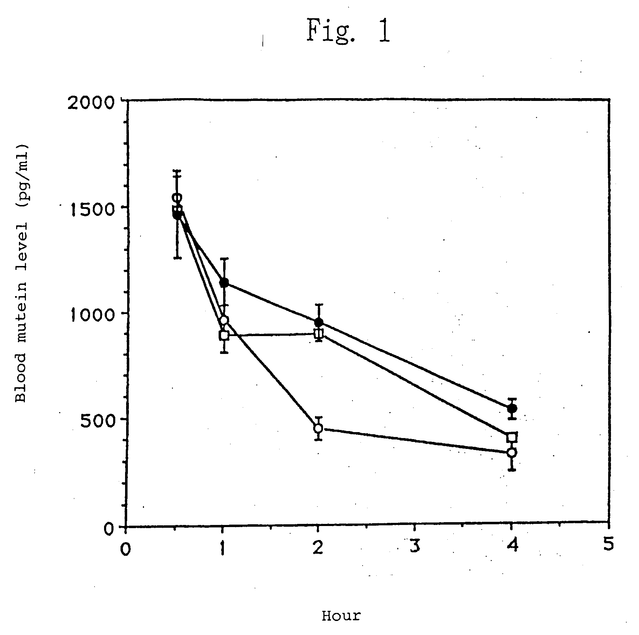

The injection of Example 1 was subcutaneously administered to 8-week-old male SD rats on their back at a dose of 160 micrograms/kilogram. As controls, Comparative Preparation 1 and Comparative Preparation 2 were administered in the same manner. Before administration and at regular intervals after administration, about 0.4-milliliter portions of blood were collected. The serum was separated from each blood sample and assayed for serum rhbFGF mutein CS23 level by ELISA. The results obtained are shown in Fig. 1. The figure graphically shows the courses of change in blood mutein level after administration of Comparative Preparation 1(o), Comparative Preparation 2(□), and the rhbFGF mutein CS23 injection of Example 1 (●), respectively. Rats were used in groups of 5. In each graph, each dot indicates a mean value and each bar a standard error (S.E.).

It was found that while rhbFGF mutein CS23 rapidly disappeared from the blood in the group given Comparative Preparation 1 (simple rhbFGF mutein CS23 solution), the blood rhbFGF mutein CS23 level was maintained high in the group given Comparative Preparaion 2 containing hyaluronic acid owing to the effect of hyaluronic acid. It was found, however, that the blood mutein level was maintained still higher at hour 1 and hour 4 after administration in the group given the injection of Example 1 prepared by adding hyaluronic acid and serum albumin to rhbFGF mutein CS23 as compared with the group given Comparative Preparation 2 prepared by adding hyaluronic acid alone.

### Experimental Example 2

The following two injections were further prepared and subjected to testing.

### Comparative Preparation 3

Three milligrams of pig insulin (26.8 units/milligram; Diosynth) was dissolved in 1.5 milliliters of 0.1 N hydrochloric acid and then 1.65 milliliters of physiological saline for injection was added, followed by addition of 15.75 milligrams of sodium hyaluronate (average molecular weight 1,470,000 daltons; Genzyme).

### Comparative Preparation 4

### Physiological saline for injection 2 milliliters

The insulin injection of Example 36 and Comparative Preparation 3 were respectively administered subcutaneously to 8-week-old male SD rats on their back at a dose of 200 micrograms/rat. In a control group, Comparative Preparation 4, namely physiological saline for injection, was administered at the same voluminal dose as used in administering the preparation of Example 36 and Comparative Preparation 3. Before administration and at regular intervals after administration, 0.5-milliliter portions of blood were collected and the serum samples derived therefrom were assayed for serum glucose level using a glucose C test kit (Wako Pure Chemical, Japan).

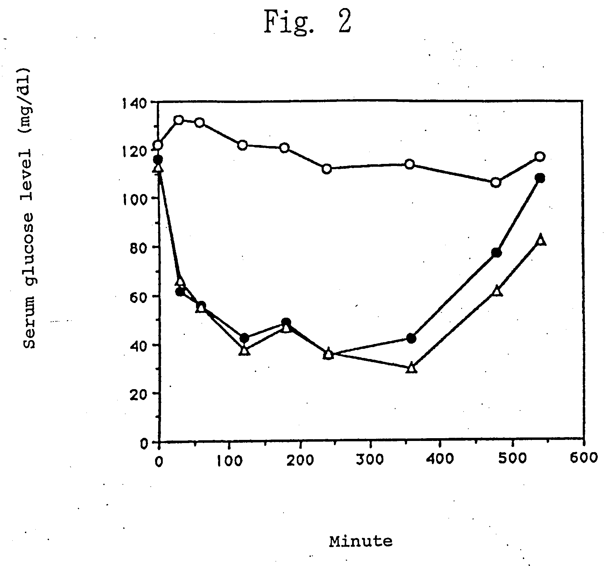

The results obtained are shown in Fig. 2. The figure shows the time courses of the serum glucose level as respectively revealed in the group given Comparative Preparation 4 (physiological saline) (o), the group given Comparative Preparation 3 (solution of pig insulin plus hyaluronic acid) (o colored black) and in the group given the solution of Example 36 containing pig insulin plus a combination of hyaluronic acid and serum albumin (△). Rats were used in groups of 5 and, in Fig. 2, each dot indicates a mean value. In the group given Comparative Preparation 4 (physiological saline), the serum glucose level remained almost unchanged. In the groups given Comparative Preparation 3 and the preparation Example 36, respectively, the serum glucose level was suppressed to substantially the same extent until minute 240 postadministration. At minute 360 postadministration and thereafter, the serum glucose level reduction lasted for a longer period in the group given the preparation of Example 36. At minute 540 postadministration, the serum glucose level had substantially returned to a normal value range in the Comparative Preparation 3 group while, in the Example 36 group, the level was still about two thirds of that in the physiological saline (Comparative Preparation 4) group. This indicates that while the combination of insulin and hyaluronic acid is said to prolong the effect of the peptide, further addition of serum albumin to this system can cause the pharmacological effect to last still longer at the same dose of insulin and in the same amount of hyaluronic acid.

### Experimental Example 3

The following injections were further prepared and tested.

### Comparative Preparation 5

To 0.0233 milliliter of a human granulocyte colony stimulating factor preparation (Nupogen; Amgen) containing, per milliliter, 300 micrograms of a recombinant human G-CSF, 50 milligrams of mannitol and 0.004% of Tween® 80 was added 1.377 milliliters of physiological saline for injection. To the resultant mixture was added 0.7 milliliter of a 1.5% (w/v) solution of sodium hyaluronate (average molecular weight: 1,470,000 daltons; Genzyme) in physiological saline (Fuso Pharmaceutical). A glass vial (capacity: 5 milliliters) containing these solutions was hermetically stoppered and subjected to rotary blending in a 200-milliliter eggplant-shaped flask equipped with a three-one motor (Heydon) by rotation (20-100 rpm) about its longitudinal axis for one hour. The above procedure gave a long-acting dosing solution substantially free of bubbles.

### Comparative Preparation 6

To 0.0233 milliliter of a human granulocyte colony stimulating factor preparation (Nupogen; Amgen) containing, per milliliter, 300 micrograms of a recombinant human G-CSF, 50 milligrams of mannitol and 0.004% of Tween® 80 was added 2.077 milliliters of physiological saline for injection.

### Comparative Preparation 7

To 1.4 milliliters of physiological saline for injection was added 0.7 milliliter of a 1.5% (w/v) solution of sodium hyaluronate (average molecular weight: 1,470,000 daltons; Genzyme) in physiological saline for injection (Fuso Pharmaceutical). A glass vial (capacity: 5 milliliters) containing these solutions was hermetically closed and subjected to rotary blending in a 200-milliliter eggplant-shaped flask equipped with a three-one motor (Heydon) by rotation (20-100 rpm) about its longitudinal axis for one hour. The above procedure gave a long-acting dosing solution substantially free of bubbles.

Eight-week-old male SD rats were subcutaneously dosed by injection at their back, with the human G-CSF-containing preparation of Example 39, Comparative Preparation 5 or Comparative Preparation 6 in a volume of 0.3 milliliter. In a control group, the solution of sodium hyaluronate in physiological saline for injection (Comparative Preparation 7) was administered in a volume of 0.3 milliliter. Before administration and serially after administration, about 0.1 milliliter of blood was withdrawn (EDTA·2Na used as anticoagulant). Peripheral leukocytes, erythrocytes and platelets were counted using a CC-180A microcell counter (Toa Iyo Denshi, Japan). Peripheral neutrophil, lymphocyte, monocyte and eosinophil counts were estimated by multiplying the respective cell occurrence frequencies, as found by typing, under a microscope, of 200 leukocytes for each Giemsa-stained smear preparation, by the leukocyte count.

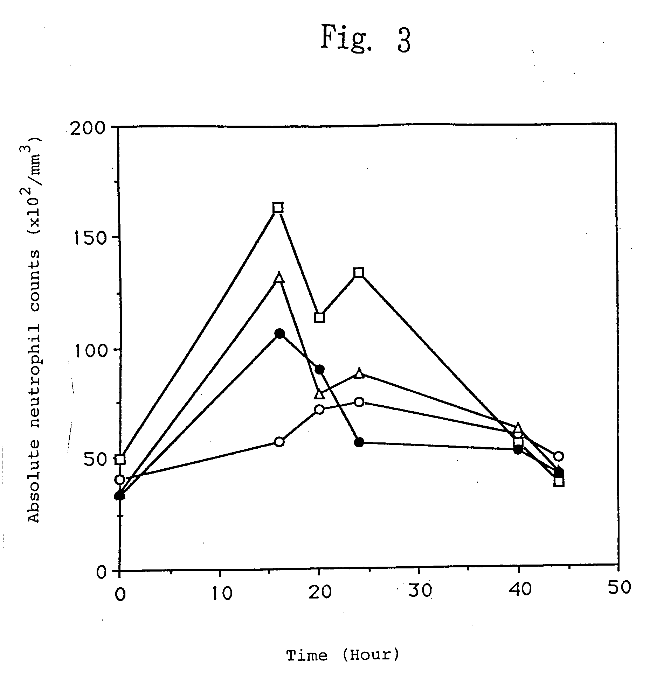

The results thus obtained are shown in Fig. 3. In the control group (o) given the aqueous hyaluronic acid solution (Comparative Preparation 7), the peripheral blood neutrophil count changed little. In the positive control group (o colored black) given the human G-CSF solution (Comparative Preparation 6), the peripheral blood 16 hours after administration showed a nearly doubled neutrophil count as compared with the control group. In the group (△) given Comparative Preparation 5, which contained hyaluronic acid additionally, the peripheral blood showed a further increase in neutrophil count. In the group (□) given the preparation of Example 39, which contained human G-CSF in combination with hyaluronic acid plus human serum albumin, the peripheral blood showed a still further increase in neutrophil count as compared with the group given Comparative Preparation 5. At 24 hours after administration, the peripheral blood neutrophil counts in the groups given Comparative Preparation 5 and Comparative Preparation 6, respectively, were already at substantially the same level as in the control group whereas, in the group given the preparation of Example 39, the neutrophil count was still approximately twice the level in the control group. These results indicate that the addition of human serum albumin can further potentiate the pharmacological effect prolonging action of hyaluronic acid. In the figure, each point indicates a mean of 5 rats.

### Experimental example 4

The following injection was further prepared and tested.

### Comparative Preparation 8

To each of two vials of Canferon-A300 (Takeda Chemical, Japan) containing 3 million international units (IU) of interferon alfa-2a and 5 mg of human serum albumin was added 1 ml of distilled water to provide an interferon alfa injection. 1.4 ml of the interferon alfa injection prepared as above (containing 4.2 million IU of interferon alfa-2a) and 0.7 ml of physiological saline for injection were put into a glass vial (capacity: 5 ml) and mixed gently.

Eight-week-old male SD rats were subcutaneously dosed by injection at their back, with the interferon alfa-2a-containing preparation of Example 49 or Comparative Preparation 8 in volume of 0.3 milliliter. Before administration and serially after administration, 0.4 ml portions of blood were collected. The serum was separated from each blood sample and assayed for serum interferon alfa 2-a level by ELISA.

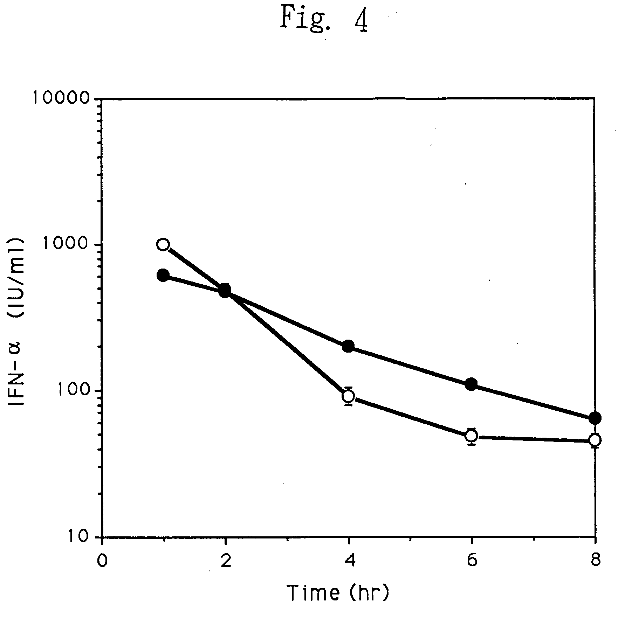

The results are plotted in Fig. 4. Interferon alfa 2-a was rapidly absorbed from the injection site in the control group treated with Comparative Preparation 8 and the serum interferon alfa 2-a concentration fell with time (o). On the other hand, in the group treated with the preparation of Example 49 containing hyaluronic acid, human serum albumin, and interferon alfa 2-a, the serum interferon alfa 2-a concentrations were higher compared with those of the control group from 1 hour till 8 hours after administration (●). These results manifest the positive effect of the combination of hyaluronic acid, human serum albumin, and interferon alfa 2-a in sustaining the serum drug concentrations. Each point represents the mean of five rats.

### Experimental Example 5

The following composition was further prepared and tested.

### Comparative Preparation 9

To 0.4 milliliter of a solution of recombinant human basic FGF mutein CS23(EP-281,822) (0.96 milligram protein/milliliter) was added 3.6 milliliters of physiological saline for injection.

Eight-week-old male SD rats fasted for 24 hours were lightly anesthetized by intraperitoneal injection of sodium pentobarbital (Somnopentyl, Pitman Moore, U.S.A.) and were orally administered 1 milliliter of either the preparation of Example 50 or the preparation of Comparative Preparation 9 to stomach. At 30 minutes post administration, incision was made at abdomen and the content of the stomach was collected. Portion of the collected content was immediately centrifuged (Microfuge B, Beckman, U.S.A.), and a 50 microliters aliquot of the supernatant was mixed with a 100 microliters aliquot of a protease inhibitor (Aprotinin, Sigma, U.S.A.) and stored at -40°C for further determination of CS23 concentration by enzyme immno assay.

| | CS23 Concentration (pg/ml) |
|---|---|
| Comparative Preparation 9 | 1768 |
| Preparation of Example 50 | 8448 |

The results manifest the positive effect of the combination of hyaluronic acid, human serum albumin, and recombinant human basic FGF mutein CS23, in persisting in succus gastricus inside stomach.

### Experimental Example 6

The following composition was further prepared and tested.

### Comparative Preparation 10

In 2 ml of physiological saline for injection (Fuso Pharmaceutical) was added 300 micrograms of parathyroid hormone (Bachem Fine Chemicals).

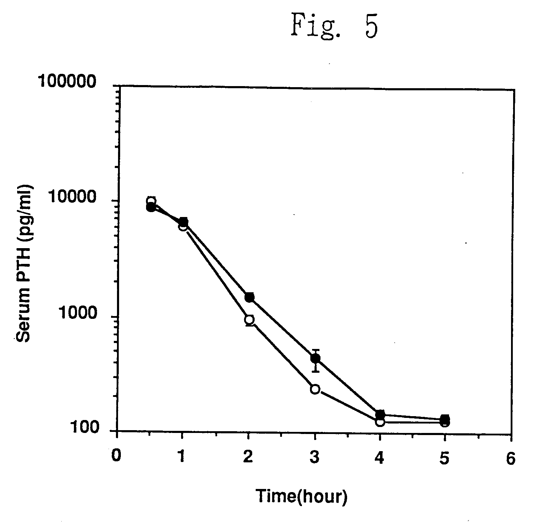

The composition of Example 51 (●) and Comparative Preparation 10 (o) were respectively administered subcutaneously at the back of 8-week-old male SD rats in a volume of 0.2 ml. Before administration and serially after administration, 0.4 ml of blood was withdrawn and serum sample was separated for the determination of PTH by radio immunoassay. As shown in Fig. 5, at 2 hours and 3 hours post administration the serum PTH level was significantly higher in the group of given Example 51 than in the group given Comparative Preparation 10.

The results manifest the positive effect of the combination of hyaluronic acid, human serum albumin, and PTH in prolonging the serum level of PTH.

## Claims (Claims for the following Contracting State(s): AT, BE, CH, DE, DK, FR, GB, IT, LI, LU, NL, PT, SE)

1. A water-soluble composition which comprises
(a) a pharmacologically active polypeptide having a molecular weight of 200 to 200 000, being secreted by an animal body and being selected from cytokines, peptide hormones, growth factors, factors affecting the cardiovascular system, factors affecting the central and peripheral nerve system, factors affecting body fluid electrolytes and blood organic constituents, factors affecting the bone and skeletal system, factors affecting the gastro-intestinal system, factors affecting the immune system, factors affecting the respiratory system, factors affecting the genital system, muteins, derivatives, analogs, homologs and active fragments thereof, except erythropoietin, or
a chemically synthesized pharmacologically active substance;
(b) a water-soluble species of hyaluronic acid or its non-toxic salt; and
(c) a water-soluble protein injectable into body fluids without showing any substantial pharmacological activity,
wherein said composition sustains the release of the ingredient (a).

2. The water-soluble composition as claimed in claim 1, wherein the pharmacologically active polypeptide is selected from interferons, interleukins, granulocyte colony stimulating factor (G-CSF), granulocyte-macrophage colony stimulating factor (GM-CSF), macrophage colony stimulating factor (M-CSF), fibroblast growth factor (FGF), tumor necrotizing factor (TNF), parathyroid hormone (PTH), calcitonin, insulin or luteinizing hormone-releasing hormone (LH-RH).

3. A water-soluble composition as claimed in claim 1, wherein the chemically synthesized pharmacologically active substance is selected from substances affecting the cardiovascular system, substances affecting the cell attachment, substances affecting the central and peripheral nerve system, substances affecting body fluid electrolytes and blood organic constituents, substances affecting the bone and skeletal system, substances affecting the gastro-intestinal system, substances affecting the immune system and substances affecting infective microorganisms.

4. The water-soluble composition as claimed in claim 1, wherein the water-soluble protein is human serum albumin.

5. The water-soluble composition as claimed in claim 1, which shows a viscosity of not more than 500 mPa·s (cp) in an aqueous solution form.

6. The water-soluble composition as claimed in claim 1, which is in the form of preparation for parenteral administration.

7. The water-soluble composition as claimed in claim 1, which is in the form of an injectable preparation.

8. The water-soluble composition as claimed in claim 1, wherein the weight ratio of the pharmacologically active substance to hyaluronic acid or its non-toxic salt is in the range of 0.0001 : 1 to 10 : 1, and the weight ratio of water-soluble protein to hyaluronic acid or its non-toxic salt is in the range of 0.001 : 1 to 100 : 1.

## Claims (Claims for the following Contracting State(s): ES, GR)

1. A method for preparing a water-soluble composition, which comprises mixing
(a) a pharmacologically active polypeptide having a molecular weight of 200 to 200 000, being secreted by an animal body and being selected from cytokines, peptide hormones, growth factors, factors affecting the cardiovascular system, factors affecting the central and peripheral nerve system, factors affecting body fluid electrolytes and blood organic constituents, factors affecting the bone and skeletal system, factors affecting the gastro-intestinal system, factors affecting the immune system, factors affecting the respiratory system, factors affecting the genital system, muteins, derivatives, analogs, homologs and active fragments thereof, except erythropoietin, or
a chemically synthesized pharmacologically active substance;
(b) a water-soluble species of hyaluronic acid or its non-toxic salt; and
(c) a water-soluble protein injectable into body fluids without showing any substantial pharmacological activity,
whereby the prepared composition sustains the release of the ingredient (a).

2. The method as claimed in claim 1, wherein the pharmacologically active polypeptide is selected from interferons, interleukins, granulocyte colony stimulating factor (G-CSF), granulocyte-macrophage colony stimulating factor (GM-CSF), macrophage colony stimulating factor (M-CSF), fibroblast growth factor (FGF), tumor necrotizing factor (TNF), parathyroid hormone (PTH), calcitonin, insulin or luteinizing hormone-releasing hormone (LH-RH).

3. The method as claimed in claim 1, wherein the chemically synthesized pharmacologically active substance is selected from substances affecting the cardiovascular system, substances affecting the cell attachment, substances affecting the central and peripheral nerve system, substances affecting body fluid electrolytes and blood organic constituents, substances affecting the bone and skeletal system, substances affecting the gastro-intestinal system, substances affecting the immune system and substances affecting infective microorganisms.

4. The method as claimed in claim 1, wherein the water-soluble protein is human serum albumin.

5. The method as claimed in claim 1, wherein the prepared composition shows a viscosity of not more than 500 mPa·s (cp) in an aqueous solution form.

6. The method as claimed in claim 1, wherein the prepared composition is in the form of preparation for parenteral administration.

7. The method as claimed in claim 1, wherein the prepared composition is in the form of an injectable preparation.

8. The method as claimed in claim 1, wherein the weight ratio of the pharmacologically active substance to hyaluronic acid or its non-toxic salt is in the range of 0.0001 : 1 to 10 : 1, and the weight ratio of water-soluble protein to hyaluronic acid or its non-toxic salt is in the range of 0.001 : 1 to 100 : 1.

## Patentansprüche (Patentansprüche für folgende(n) Vertragsstaat(en): AT, BE, CH, DE, DK, FR, GB, IT, LI, LU, NL, PT, SE)

1. Wasserlösliche Zusammensetzung, umfassend:
(a) ein pharmakologisch aktives Polypeptid mit einem Molekulargewicht von 200 bis 200 000, das durch einen Tierkörper sezerniert wird und das aus Cytokinen, Peptidhormonen, Wachstumsfaktoren, Faktoren, die das cardiovaskuläre System beeinflussen, Faktoren, die das zentrale und periphere Nervensystem beeinflussen, Faktoren, die Elektrolyte in Körperflüssigkeiten und organische Bestandteile des Blutes beeinflussen, Faktoren, die das Knochen- und Skelettsystem beeinflussen, Faktoren, die das gastrointestinale System beeinflussen, Faktoren, die das Immunsystem beeinflussen, Faktoren, die das respiratorische System beeinflussen, Faktoren, die das Genitalsystem beeinflussen, Muteinen, Derivaten, Analoga, Homologen und aktiven Fragmenten davon außer Erythropoietin ausgewählt ist, oder eine chemisch synthetisierte pharmakologisch aktive Substanz;
(b) eine wasserlösliche Spezies von Hyaluronsäure oder einem nichttoxischen Salz davon; und
(c) ein wasserlösliches Protein, das in Körperflüssigkeiten injizierbar ist, ohne eine wesentliche pharmakologische Wirkung zu zeigen;
wobei die Zusammensetzung die Freisetzung des Bestandteils (a) verzögert.

2. Wasserlösliche Zusammensetzung gemäß Anspruch 1, wobei das pharmakologisch aktive Polypeptid aus Interferonen, Interleukinen, Granulocyten-koloniestimulierendem Faktor (G-CSF), Granulocyten-Makrophagen-koloniestimulierendem Faktor (GM-CSF), Makrophagen-koloniestimulierendem Faktor (M-CSF), Fibroblasten-Wachstumsfaktor (FGF), Tumornekrosefaktor (TNF), Parathyroidhormon (PTH), Calcitonin, Insulin oder luteinisierendes-Hormon-freisetzendem Hormon (LH-RH) ausgewählt ist.

3. Wasserlösliche Zusammensetzung gemäß Anspruch 1, wobei die chemisch synthetisierte pharmakologisch aktive Substanz aus Substanzen, die das cardiovaskuläre System beeinflussen, Substanzen, die die Zellanlagerung beeinflussen, Substanzen, die das zentrale und periphere Nervensystem beeinflussen, Substanzen, die Elektrolyte in Körperflüssigkeiten und organische Bestandteile des Blutes beeinflussen, Substanzen, die das Knochen- und Skelettsystem beeinflussen, Substanzen, die das gastrointestinale System beeinflussen, Substanzen, die das Immunsystem beeinflussen, und Substanzen, die infektiöse Mikroorganismen beeinflussen, ausgewählt ist.

4. Wasserlösliche Zusammensetzung gemäß Anspruch 1, wobei es sich bei dem wasserlöslichen Protein um Humanserumalbumin handelt.

5. Wasserlösliche Zusammensetzung gemäß Anspruch 1, die in Form einer wäßrigen Lösung eine Viskosität von nicht mehr als 500 mPa·s (cP) aufweist.

6. Wasserlösliche Zusammensetzung gemäß Anspruch 1, die in Form eines Präparats für die parenterale Verabreichung vorliegt.

7. Wasserlösliche Zusammensetzung gemäß Anspruch 1, die in Form eines injizierbaren Präparats vorliegt.

8. Wasserlösliche Zusammensetzung gemäß Anspruch 1, wobei das Gewichtsverhältnis von pharmakologisch aktiver Substanz zu Hyaluronsäure oder ihrem nichttoxischen Salz im Bereich von 0,0001:1 bis 10:1 liegt und das Gewichtsverhältnis von wasserlöslichem Protein zu Hyaluronsäure oder ihrem nichttoxischen Salz im Bereich von 0,001:1 bis 100:1 liegt.

## Patentansprüche (Patentansprüche für folgende(n) Vertragsstaat(en): ES, GR)

1. Verfahren zur Herstellung einer wasserlöslichen Zusammensetzung, umfassend das Mischen:
(a) eines pharmakologisch aktiven Polypeptids mit einem Molekulargewicht von 200 bis 200 000, das durch einen Tierkörper sezerniert wird und das aus Cytokinen, Peptidhormonen, Wachstumsfaktoren, Faktoren, die das cardiovaskuläre System beeinflussen, Faktoren, die das zentrale und periphere Nervensystem beeinflussen, Faktoren, die Elektrolyte in Körperflüssigkeiten und organische Bestandteile des Blutes beeinflussen, Faktoren, die das Knochen- und Skelettsystem beeinflussen, Faktoren, die das gastrointestinale System beeinflussen, Faktoren, die das Immunsystem beeinflussen, Faktoren, die das respiratorische System beeinflussen, Faktoren, die das Genitalsystem beeinflussen, Muteinen, Derivaten, Analoga, Homologen und aktiven Fragmenten davon außer Erythropoietin ausgewählt ist, oder einer chemisch synthetisierten pharmakologisch aktiven Substanz;
(b) einer wasserlöslichen Spezies von Hyaluronsäure oder einem nichttoxischen Salz davon; und
(c) eines wasserlöslichen Proteins, das in Körperflüssigkeiten injizierbar ist, ohne eine wesentliche pharmakologische Wirkung zu zeigen;
wobei die hergestellte Zusammensetzung die Freisetzung des Bestandteils (a) verzögert.

2. Verfahren gemäß Anspruch 1, wobei das pharmakologisch aktive Polypeptid aus Interferonen, Interleukinen, Granulocyten-koloniestimulierendem Faktor (G-CSF), Granulocyten-Makrophagen-koloniestimulierendem Faktor (GM-CSF), Makrophagen-koloniestimulierendem Faktor (M-CSF), Fibroblasten-Wachstumsfaktor (FGF), Tumornekrosefaktor (TNF), Parathyroidhormon (PTH), Calcitonin, Insulin oder luteinisierendes-Hormon-freisetzendem Hormon (LH-RH) ausgewählt ist.

3. Verfahren gemäß Anspruch 1, wobei die chemisch synthetisierte pharmakologisch aktive Substanz aus Substanzen, die das cardiovaskuläre System beeinflussen, Substanzen, die die Zellanlagerung beeinflussen, Substanzen, die das zentrale und periphere Nervensystem beeinflussen, Substanzen, die Elektrolyte in Körperflüssigkeiten und organische Bestandteile des Blutes beeinflussen, Substanzen, die das Knochen- und Skelettsystem beeinflussen, Substanzen, die das gastrointestinale System beeinflussen, Substanzen, die das Immunsystem beeinflussen, und Substanzen, die infektiöse Mikroorganismen beeinflussen, ausgewählt ist.

4. Verfahren gemäß Anspruch 1, wobei es sich bei dem wasserlöslichen Protein um Humanserumalbumin handelt.

5. Verfahren gemäß Anspruch 1, wobei die hergestellte Zusammensetzung in Form einer wäßrigen Lösung eine Viskosität von nicht mehr als 500 mPa·s (cP) aufweist.

6. Verfahren gemäß Anspruch 1, wobei die hergestellte Zusammensetzung in Form eines Präparats für die parenterale Verabreichung vorliegt.

7. Verfahren gemäß Anspruch 1, wobei die hergestellte Zusammensetzung in Form eines injizierbaren Präparats vorliegt.

8. Verfahren gemäß Anspruch 1, wobei das Gewichtsverhältnis von pharmakologisch aktiver Substanz zu Hyaluronsäure oder ihrem nichttoxischen Salz im Bereich von 0,0001:1 bis 10:1 liegt und das Gewichtsverhältnis von wasserlöslichem Protein zu Hyaluronsäure oder ihrem nichttoxischen Salz im Bereich von 0,001:1 bis 100:1 liegt.

## Revendications (Revendications pour l'(les) Etat(s) contractant(s) suivant(s): AT, BE, CH, DE, DK, FR, GB, IT, LI, LU, NL, PT, SE)

1. Composition hydrosoluble qui comprend :
(a) un polypeptide pharmacologiquement actif présentant une masse moléculaire de 200 à 200 000, étant secrété par un corps animal et étant choisi parmi les cytokines, les hormones peptidiques, les facteurs de croissance, les facteurs affectant le système cardiovasculaire, les facteurs affectant le système nerveux périphérique et central, les facteurs affectant les électrolytes des fluides corporels et les constituants organiques du sang, les facteurs affectant le système osseux et squelettique, les facteurs affectant le système gastro-intestinal, les facteurs affectant le système immunitaire, les facteurs affectant le système respiratoire, les facteurs affectant le système génital, les mutéines, les composés dérivés, analogues, homologues et les fragments actifs de ces derniers, à l'exception de l'érythropoïétine, ou une substance pharmacologiquement active synthétisée par voie chimique ;
(b) une espèce hydrosoluble d'acide hyaluronique ou son sel non-toxique ; et
(c) une protéine hydrosoluble injectable dans les fluides corporels, qui ne présente aucune quelconque activité pharmacologique importante,
dans laquelle ladite composition permet une libération prolongée de l'ingrédient (a).

2. Composition hydrosoluble selon la revendication 1, dans laquelle le polypeptide pharmacologiquement actif est choisi parmi les interférons, les interleukines, le facteur de stimulation des colonies de granulocytes (G-CSF), le facteur de stimulation des colonies de granulocytes et de macrophages (GM-CSF), le facteur de stimulation des colonies de macrophages (M-CSF), le facteur de croissance des fibroblastes (FGF), le facteur de nécrose des tumeurs (TNF), l'hormone parathyroïdienne (PTH), la calcitonine, l'insuline ou l'hormone libératrice de la lutéinostimuline (LH-RH).

3. Composition hydrosoluble selon la revendication 1, dans laquelle la substance pharmacologiquement active synthétisée par voie chimique est choisie parmi les substances affectant le système cardiovasculaire, les substances affectant l'ancrage des cellules, les substances affectant le système nerveux central et périphérique, les substances affectant les électrolytes des fluides corporels et les constituants organiques du sang, les substances affectant le système osseux et squelettique, les substances affectant le système gastro-intestinal, les substances affectant le système immunitaire et les substances affectant les micro-organismes infectieux.

4. Composition hydrosoluble selon la revendication 1, dans laquelle la protéine hydrosoluble est l'albumine sérique humaine.

5. Composition hydrosoluble selon la revendication 1, que présente une viscosité ne dépassant pas 500 mPa.s (cp) sous une forme de solution aqueuse.

6. Composition hydrosoluble selon la revendication 1, qui se trouve sous la forme d'une préparation pour une administration par voie parentérale.

7. Composition hydrosoluble selon la revendication 1, qui se trouve sous la forme d'une préparation injectable.

8. Composition hydrosoluble selon la revendication 1, dans laquelle le rapport pondéral de la substance pharmacologiquement active à l'acide hyaluronique ou son sel non-toxique se situe dans l'intervalle allant de 0,0001:1 à 10:1, et le rapport pondéral de la protéine hydrosoluble à l'acide hyaluronique ou son sel non-toxique se situe dans l'intervalle allant de 0,001:1 à 100:1.

## Revendications (Revendications pour l'(les) Etat(s) contractant(s) suivant(s): ES, GR)

1. Procédé pour préparer une composition hydrosoluble, qui comprend le mélangeage de :
(a) un polypeptide pharmacologiquement actif présentant une masse moléculaire de 200 à 200 000, étant secrété par un corps animal et étant choisi parmi les cytokines, les hormones peptidiques, les facteurs de croissance, les facteurs affectant le système cardiovasculaire, les facteurs affectant le système nerveux périphérique et central, les facteurs affectant les électrolytes des fluides corporels et les constituants organiques du sang, les facteurs affectant le système osseux et squelettique, les facteurs affectant le système gastro-intestinal, les facteurs affectant le système immunitaire, les facteurs affectant le système respiratoire, les facteurs affectant le système génital, les mutéines, les composés dérivés, analogues, homologues et les fragments actifs de ces derniers, à l'exception de l'érythropoïétine, ou une substance pharmacologiquement active synthétisée par voie chimique ;
(b) une espèce hydrosoluble d'acide hyaluronique ou son sel non-toxique ; et
(c) une protéine hydrosoluble injectable dans les fluides corporels, qui ne présente aucune quelconque activité pharmacologique importante,
la composition préparée permettant une libération soutenue de l'ingrédient (a).

2. Procédé selon la revendication 1, dans lequel le polypeptide pharmacologiquement actif est choisi parmi les interférons, les interleukines, le facteur de stimulation des colonies de granulocytes (G-CSF), le facteur de stimulation des colonies de granulocytes et de macrophages (GM-CSF), le facteur de stimulation des colonies de macrophages (M-CSF), le facteur de croissance des fibroblastes (FGF), le facteur de nécrosant des tumeurs (TNF), l'hormone parathyroïdienne (PTH), la calcitonine, l'insuline ou l'hormone libératrice de la lutéinostimuline (LH-RH).

3. Procédé selon la revendication 1, dans lequel la substance pharmacologiquement active synthétisée par voie chimique est choisie parmi les substances affectant le système cardiovasculaire, les substances affectant l'ancrage des cellules, les substances affectant le système nerveux central et périphérique, les substances affectant les électrolytes des fluides corporels et les constituants organiques du sang, les substances affectant le système osseux et squelettique, les substances affectant le système gastro-intestinal, les substances affectant le système immunitaire, les facteurs affectant les micro-organismes infectieux.

4. Procédé selon la revendication 1, dans lequel la protéine hydrosoluble est l'albumine sérique humaine.

5. Procédé selon la revendication 1, dans lequel la composition préparée présente une viscosité ne dépassant pas 500 mPa.s (cp) sous une forme de solution aqueuse.

6. Procédé selon la revendication 1, dans lequel la composition préparée se trouve sous la forme d'une préparation pour une administration par voie parentérale.

7. Procédé selon la revendication 1, dans lequel la composition préparée se trouve sous la forme d'une préparation injectable.

8. Procédé selon la revendication 1, dans lequel le rapport pondéral de la substance pharmacologiquement active à l'acide hyaluronique ou son sel non-toxique se situe dans l'intervalle allant de 0,0001:1 à 10:1, et le rapport pondéral de la protéine hydrosoluble à l'acide hyaluronique ou son sel non-toxique se situe dans l'intervalle allant de 0,001:1 à 100:1.
